# EUROPEAN PATENT SPECIFICATION

(11) **EP 4 169 454 B1**
(45) Date of publication and mention of the grant of the patent: **28.01.2026**
(21) Application number: 21828922.1
(22) Date of filing: 10.06.2021
(51) Int. Cl.: A61B 17/00, B33Y 80/00

(54) **CLOSURE SYSTEM**
VERSCHLUSSSYSTEM
SYSTÈME DE FERMETURE

(30) Priority: 22.06.2020 JP 2020107299
(43) Date of publication of application: 26.04.2023
(73) Proprietor: Netbusiness Consulting Co., Ltd., Tokyo, 104-0053 (JP)
(72) Inventor: UCHIDA, Harutoshi, Tokyo 104-0053 (JP)
(74) Representative: Hoffmann Eitle
(86) International application number: PCT/JP2021/022181
(87) International publication number: WO 2021/261279

(56) References cited:
- WO-A1-2018/213230
- WO-A2-2008/071952
- JP-A- 2003 512 128
- JP-A- 2007 504 915
- JP-A- 2008 512 139
- JP-A- 2015 500 665
- JP-A- 2020 516 356
- JP-A- S51 115 088
- US-A- 5 904 703
- US-A- 5 904 703
- US-A1- 2006 224 183
- US-A1- 2007 293 891
- US-A1- 2017 035 435
- US-A1- 2018 338 824
- US-A1- 2019 175 191
- US-B1- 6 726 696

## Description

### BACKGROUND

### Field

An embodiment of the present disclosure relates to a closure system comprising a closure device for closing a hole or duct in a tissue related to a cardiac disease. In addition, an embodiment of the present disclosure relates to a manufacturing method of a closure device.

### Background Art

In cardiac diseases such as atrial septal defect, ventricular septal defect, patent ductus arteriosus, patent foramen ovale, etc., a hole or duct is formed in a tissue such as atrial septum, pulmonary artery, etc. A closure device is used as a tool for closing the hole or duct. For example, Patent Literature 1 discloses a method of closing a hole using a mesh structure formed of woven metal wires. When pushed out from a catheter inserted in a heart, the mesh structure can expand like a disc in the heart.

### Patent Literature

Patent Literature 1: JP 6661539 B

### Non Patent Literature

Non patent Literature 1: Zahid Amin, Echocardiographic Predictors of Cardiac Erosion After Amplatzer Septal Occluder Placement, Catheterization and Cardiovascular Interventions (2014) 83:84-92
Non Patent Literature 2: Tadaaki Abe; Shinya Tsukano; Yuko Tosaka, Pericardial tamponade due to erosion of a Figulla Flex II device after closure of an atrial septal defect, Catheter Cardiovasc Interv. (2019) 94:1003-1005.
Non Patent Literature 3: Masataka Kitano; Satoshi Yazaki; Hisashi Sugiyama; Shin-ichi Ohtsuki; Hideshi Tomita, Risk Factors and Predictors of Cardiac Erosion Discovered from 12 Japanese Patients Who Developed Erosion After Atrial Septal Defect Closure Using Amplatzer Septal Occluder, Pediatric Cardiology (2020) 41:297-308
Non Patent Literature 4: Preetham Kumar; James L. Orford; Jonathan M. Tobis, Two cases of pericardial tamponade due to nitinol wire fracture of a gore septal occluder, Catheter Cardiovasc Interv. (2020) 96:219-224.

US 5,904,703 A discloses features falling under the preamble of claim 1.

### SUMMARY

The use of the mesh structure limits a planar shape of the closure device. This may make it difficult to properly close a hole or duct, depending on a position and a shape of the hole or duct.

For example, Non Patent Literatures 1 to 4 introduce examples in which a hole of atrial septal defect is closed by using an Amplatzer septal occluder (hereinafter referred to also as ASO). The ASO comprises a mesh structure formed of woven metal wires. Non Patent Literatures 1 to 3 report a mechanical damage (referred to also as erosion), such as cardiac perforation formed in a tissue, which is caused by contact with the ASO. Non Patent Literature 4 reports that some of the metal wires in the mesh structure were damaged, which results in erosion. If breeding or the like occurs because of the erosion, there is a risk of cardiac tamponade. The cardiac tamponade is a state in which a heart is unable to sufficiently dilate due to increase in inner pressure of cardiac sac caused by increase in cardiac sac fluid.

The object of the present disclosure is to provide a closure device and a closure system capable of effectively solving such a problem.

The invention is defined by claim 1.

An embodiment of the present disclosure is a closure system for closing a hole or duct in a tissue related to a cardiac disease, comprising:
a catheter having an internal diameter D1 [mm]; and
a closure device to be delivered to the hole or duct in the tissue through the catheter;
wherein:
   the closure device comprises: a first plate including a first center part, and a first extension part extending around the first center part and containing a resin having elastic restorability; and a waist part connected to the first center part;
   the elastic restorability is a property by which the first extension part returns from a state in which the first extension part is folded such that surface portions of the first extension part face to each other, to a state in which the first extension part is extended around the first center part;
   the first extension part has a thickness A1 [mm];
   the waist part has a maximum dimension T1 [mm] in a plan view; and
   the internal diameter D1 of the catheter is larger than 2×A1+T1.

In the closure system according to the present disclosure, the first extension part may have a maximum dimension B1 [mm] in a plan view, and
A1/B1 may be 1/50 or more.

In the closure system according to the present disclosure, the first plate may include a base layer extending around the first center part and containing fluororesin, polyester, polyamide, urethane, silicone, or polyetherketone, and
a thickness of the base layer may be 50% or more of the thickness A1 of the overall first plate.

In the closure system according to the present disclosure, the first extension part may have a tensile strength between 20 MPa or more and 150 MPa or less.

In the closure system according to the present disclosure, the first extension part may include, in a plan view, a first portion, and a second portion having a shape asymmetric to the first portion with respect to a center of the first center part.

The closure system according to the present disclosure may comprise a second plate connected to the waist part, wherein
the second plate may comprise a second center part connected to the waist part, and a second extension part extending around the second center part and containing a resin having elastic restorability.

In the closure system according to the present disclosure, the second extension part may have a thickness A2 [mm], and
the internal diameter D1 of the catheter may be larger than 2×A1+2×A2+T1.

The closure system according to the present disclosure comprises an IC chip embedded in the resin.

In the closure system according to the present disclosure, the IC chip includes a power generating element that generates electric power using pulses of a heart.

In the closure system according to the present disclosure, the first plate may comprise a surface layer containing a fluororesin.

In the closure system according to the present disclosure, the first plate may comprise a plurality of projections positioned on a contact surface facing the tissue.

In the closure system according to the present disclosure, the first extension part may comprise a linearly running reinforcement part.

In the closure system according to the present disclosure, the cardiac disease may be atrial septal defect, ventricular septal defect, patent ductus arteriosus, or patent foramen ovale.

The closure system according to the present disclosure may comprise a handle for operating the closure device.

In the closure system according to the present disclosure, the closure device may include a marker provided on the first plate, and
the handle may include a lever that controls a rotational angle of the first plate.

An embodiment of the present disclosure is a manufacturing method of a closure device for closing a hole or duct in a tissue related to a cardiac disease, wherein:
the closure device comprises a first plate to cover the hole or duct;
the first plate comprises a first center part, and a first extension part extending around the first center part and containing a resin having elastic restorability;
the elastic restorability is a property by which the first extension part returns from a state in which the first extension part is folded such that surface portions of the first extension part face to each other, to a state in which the first extension part is extended around the first center part;
the manufacturing method comprises a producing step that produces the closure device based on information on a shape and a position of the hole or duct in the tissue; and
the producing step comprises a first plate designing step using a learning model that is trained with a training data in such a manner that the information on the shape and the position of the hole or duct in the tissue are inputted to the learning model, and that the learning model outputs design information including any of a planar shape, a thickness, and/or a material of the first extension part, wherein the training data includes shapes and positions of holes or ducts in tissues of patients having cardiac disease as input objects, and wherein the training data includes design information including any of planar shapes, thicknesses, and/or materials of first extension parts as output values.

In the manufacturing method according to the present disclosure, the producing step may comprise a first plate producing step that produces the first plate by means of a 3D printer.

In the manufacturing method according to the present disclosure, the first extension part may have a thickness A1 [mm] and a maximum dimension B1 [mm] in a plan view, and
A1/B1 may be 1/50 or more.

In the manufacturing method according to the present disclosure, the first plate may contain fluororesin, polyester, polyamide, urethane, silicone, or polyetherketone.

In the manufacturing method according to the present disclosure, the closure device may comprise a waist part connected to the second center part, and a second plate connected to the waist part,
the second plate may comprise a second center part connected to the waist part, and a second extension part extending around the second center part and containing a resin having elastic restorability, and
the producing step may comprise a second plate designing step using a learning model that is trained with training data in such a manner that the information on the shape and the position of the hole or duct in the tissue are inputted to the learning model, and that the learning model outputs design information including any of a planar shape, a thickness, and/or a material of the second extension part, wherein the training data includes shapes and positions of holes or ducts in tissues of patients having cardiac disease as input objects, and wherein the training data includes design information including any of planar shapes, thicknesses, and/or materials of second extension parts as output values.

The present disclosure can provide a closure device and a closure system capable of appropriately various holes or ducts.

### BRIEF DESCRIPTION OF THE DRAWINGS

Fig. 1 is a view showing an example in which a duct by an arterial duct is formed between an aorta and a pulmonary artery.
Fig. 2 is a view showing that the arterial duct of Fig. 1 is closed by a closure device.
Fig. 3 is a view showing an example of the closure device.
Fig. 4 is a view illustrating elastic restorability of the closure device.
Fig. 5 is a view showing a first plate seen from a non-contact surface side.
Fig. 6 is a sectional view of the first plate of Fig. 5 seen from a VI-VI direction.
Fig. 7 is a view showing examples of materials forming a base layer of the first plate.
Fig. 8 is a view showing examples of materials forming a fiber of the first plate.
Fig. 9 is a view showing examples of materials forming a second surface layer of the first plate.
Fig. 10 is a view showing an example of a manufacturing method of the closure device.
Fig. 11A is a view showing an example of a method of closing an arterial duct using the closure device.
Fig. 11B is a view showing the example of the method of closing the arterial duct using the closure device.
Fig. 11C is a view showing the example of the method of closing the arterial duct using the closure device.
Fig. 11D is a view showing the example of the method of closing the arterial duct using the closure device.
Fig. 12 is a view showing an example of a closure system.
Fig. 13 is a view showing an example of a heart including an atrial septum in which a hole is formed.
Fig. 14 is a view showing an example of the closure system.
Fig. 15 is a view showing that the hole in the atrial septum is closed by the closure device of Fig. 14.
Fig. 16 is a view illustrating dimensions of the closure device of Fig. 14.
Fig. 17A is a view showing an example of a method of closing a hole using the closure device.
Fig. 17B is a view showing the example of the method of closing the hole using the closure device.
Fig. 17C is a view showing the example of the method of closing the hole using the closure device.
Fig. 17D is a view showing the example of the method of closing the hole using the closure device.
Fig. 18 showing an example of a heart including an atrial septum in which a hole is formed.
Fig. 19 is a view showing an example of the closure device for closing the hole of Fig. 18.
Fig. 20 is a view showing that the hole in the atrial septum of Fig. 19 is closed by the closure device.
Fig. 21 is a view showing that a hole in an interventricular septum is closed by the closure device.
Fig. 22 is a view showing an example of the closure device.
Fig. 23 is a view showing an example of a handle for operating the closure device.

### DESCRIPTION OF EMBODIMENTS

A closure device according to the present disclosure is described in detail with reference to the drawings. An embodiment described below is a mere example, and the present disclosure is not construed as limited to the embodiment. In the drawings to which the reference is made in this embodiment, the same or similar reference numeral is given to parts that are identical or have similar functions, and repeated description may be omitted. Dimensional proportions in the drawings may differ from actual proportions for the sake of explanation, and some components may be omitted from the drawings.

### (Closure System)

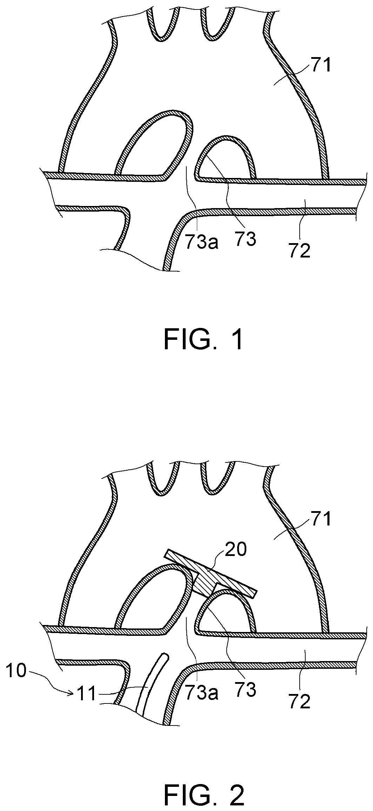

Fig. 1 shows an example of a tissue related to a cardiac disease. In the example shown in Fig. 1, the cardiac disease is patent ductus arteriosus. The patent ductus arteriosus is a condition in which an arterial duct 73 connecting an aorta 71 and a pulmonary artery 72 is not closed, so that the arterial duct 73 remains.

Fig. 2 is a view showing that the arterial duct 73 of Fig. 1 is treated by a closure system 10. The closure system 10 comprises a catheter 11 to be inserted into a body, and a closure device 20 to be delivered through the catheter 11 to the arterial duct 73. The closure device 20 is configured to close the arterial duct 73.

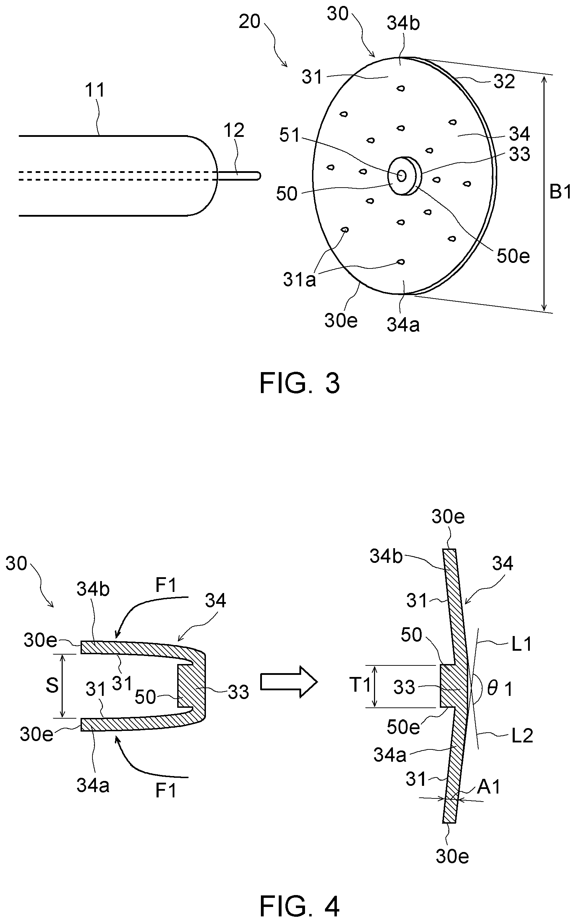

Fig. 3 is a view showing an example of the closure system 10. The closure system 10 may comprise a delivery cable 12 joined to the closure device 20. By moving the delivery cable 12 inside the catheter 11, the closure device 20 can be moved inside the catheter 11, and the closure device 20 can be pushed out from the closure device 20. After the closure device 20 has been attached to a tissue of a heart 1, the delivery cable 12 is detached from the closure device 20.

The closure device 20, which is in a folded state, is inserted into the catheter 11, and is delivered to the arterial duct 73. After having been delivered to the arterial duct 73 or the aorta 71, the closure device 20 is pushed out from the catheter 11 by the delivery cable 12. After having been pushed out from the catheter 11, the closure device 20 can unfold and elastically restore to its extending state as shown in Fig. 3.

### (Closure Device)

The closure device 20 is described in detail. The closure device 20 at least comprises a first plate 30 to cover the arterial duct 73. The closure device 20 may comprise a waist part 50 to be installed in the arterial duct 73. The waist part 50 may include a joint 51 joined to the delivery cable 12. The joint 51 includes an opening into which the delivery cable 12 is inserted, for example. The opening of the joint 51 may have a structure for facilitating attachment to the delivery cable 12 and detachment from the delivery cable 12. For example, threads may be formed on a wall surface of the opening of the joint 51.

### (First Plate)

The first plate 30 is described in detail. As shown in Fig. 3, the first plate 30 includes a contact surface 31 and a non-contact surface 32. The contact surface 31 is a surface that faces and contacts the tissue related to the cardiac disease. In this embodiment, the contact surface 31 faces and contacts a wall surface of the aorta 71. The non-contact surface 32 is a surface opposite the contact surface 31.

A shape of the first plate 30 in a plan view is described. "Plan view" means that the closure device 20 is seen along a normal direction of the contact surface 31 or the non-contact surface 32. In the example shown in Fig. 3, the first plane 30 has a circular shape in a plan view. In the description below, a shape in a plan view is referred to as planar shape.

As shown in Fig. 3, the first plate 30 includes a first center part 33, and a first extension part 34 extending around the first center part 33. The first center part 33 is connected to the waist part 50. The first center part 33 may be integral with the waist part 50. "Integral" means that there is no interface between two components. The first extension part 34 contains a resin having elastic restorability. The elastic restorability is a property by which the first extension part 34 returns from a state in which the first extension part 34 is folded such that surface portions of the first extension part 34 face to each other, to a state in which the first extension part 34 is extended around the first center part 33. The first extension part 34 may be integral with the first center part 33.

The elastic restorability is described with reference to Fig. 4.

First, as shown in the left side of Fig. 4, a force F1 is applied to the first plate 30. Thus, the first extension part 34 is folded such that the contact surface 31 of a first portion 34a of the first extension part 34 and the contact surface 31 of a second portion 34b thereof face to each other. The second portion 34b is positioned on an opposite side of the first portion 34a with respect to a center of the first center part 33. In the example shown in Figs. 3 and 4, the first portion 34a is positioned below the first center part 33, and the second portion 34b is positioned above the first center part 33. A reference numeral S of Fig. 4 represents a distance between a distal end of the first portion 34a and a distal end of the second portion 34b in the folded state. The distance S is B1/3, for example. B1 is a maximum dimension of the contact surface 31 in a plan view. When the first plate 30 is circular, the maximum dimension B1 is a diameter of the first plate 30, as shown in Fig. 3.

Following thereto, the force F1 is removed from the first plate 30. Thus, as shown in the right side of Fig. 4, the first plate 30 unfolds and elastically restores to its extending shape. In Fig. 4, θ1 represents an angle defined between a direction along which the first portion 34a unfolds and a direction along which the second portion 34b unfolds. In this patent application, when the angle θ1 is between 135° or more and 225° or less, it can be said that the first extension part 34 has elastic restorability. The angle θ1 may be between 150° or more and 210° or less.

As shown by a reference numeral L1 in Fig. 4, the direction along which the first portion 34a unfolds is defined by a direction along which the first portion 34a unfolds in a boundary between the first center part 33 and the first portion 34a. Similarly, as shown by a reference numeral L2 in Fig. 4, the direction along which the second portion 34b unfolds is defined by a direction along which the second portion 34b unfolds in a boundary between the first center part 33 and the second portion 34b. The direction L1, the direction L2, and the angle θ1 are calculated by laterally imaging the first plate 30 and by analyzing an obtained image.

The elastic restorability of the first extension part 34 can be realized by various methods. For example, the elastic restorability can be realized by appropriately setting a relationship between a thickness A1 [mm] of the first extension part 34 and the maximum dimension B1 [mm] of the first extension part 34 in a plan view. A1/B1 is, for example, 1/50 or more, may be 1/30 or more, or may be 1/20 or more. On the other hand, the first plate 30 having an excessively large thickness A1 is hard to fold, which makes it difficult to deliver the closure device 20 through the catheter 11. In consideration of this point, A1/B1 may be 1/3 or less, may be 1/5 or less, or may be 1/10 or less. As described below, the elastic restorability of the first extension part 34 may be realized based on a mechanical property such as tensile strength.

As shown in Fig. 4, in a state where the first plate 30 is extended, the waist part 50 has a maximum dimension T1 in a plan view. When the waist part 50 is circular in a plan view, the maximum dimension T1 is a diameter of the waist part 50.

As shown in Fig. 3, a plurality of projections 31a may be formed on the contact surface 31 of the first plate 30. The provision of the projections 31a can increase a contact surface area of the contact surface 31 with the wall surface of the aorta 71. This can suppress detachment of the first plate 30 from the tissue. A height, a shape, a distribution density, etc., of the projections 31a are set to improve adhesiveness of the first plate 30 to the tissue, while avoiding damage which may be given by the projection 31a to the tissue. The height of the projection 31a is, for example, 10 µm or more, may be 100 µm or more, or may be 1mm or more. The height of the projection 31a is, for example, 20 mm or less, may be 10 mm or less, or may be 5 mm or less.

In Figs. 3 and 4, a reference numeral 30e represents an outer edge of the first plate 30. The outer edge 30e is preferably free of hard material such as a metal. For example, the outer edge 30e is preferably formed of a resin such as a biocompatible material described below. This can suppress generation of erosion in the tissue, which may be caused by contact between the tissue and the outer edge 30e.

In Figs. 3 and 4, a reference numeral 50e represents an outer circumferential surface of the waist part 50. The outer circumferential surface 50e is preferably free of hard material such as a metal. For example, the outer circumferential surface 50e is preferably formed of a resin such as a biocompatible material described below. This can suppress generation of erosion in the tissue, which may be caused by contact between the tissue and the outer circumferential surface 50e.

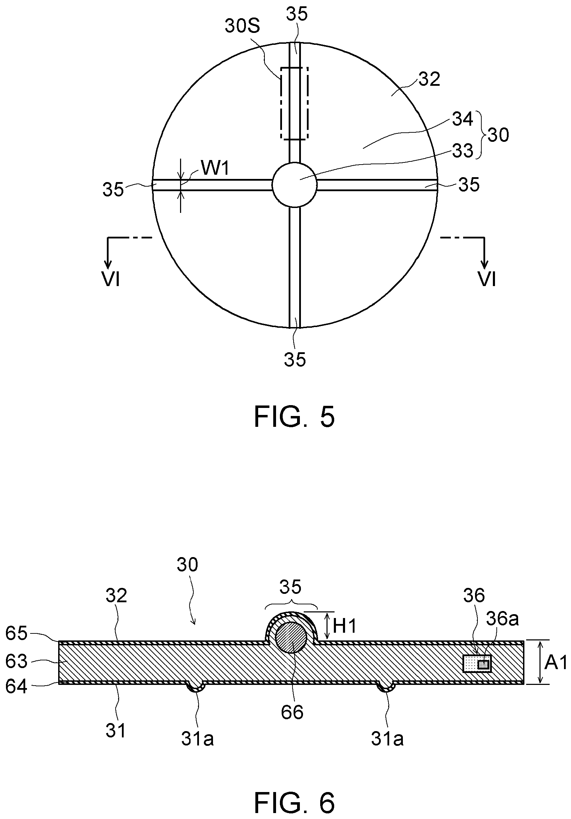

Next, a structure of the non-contact surface 32 of the first plate 30 is described. Fig. 5 is a view showing the first plate seen from the non-contact surface side. As shown in Fig. 5, a linearly running reinforcement part 35 may be formed on the non-contact surface 32. The reinforcement part 35 is raised from the non-contact surface 32. The provision of the reinforcement part 35 can improve the elastic restorability of the first extension part 34. Although not shown, the reinforcement part 35 may be formed on the contact surface 31.

As shown in Fig. 5, the reinforcement part 35 may run from the first center part 33 toward the outer edge of the first plate 30. In other words, the reinforcement part 35 may run along a radial direction of the first plate 30. Although not shown, the reinforcement part 35 may run along another direction. For example, the reinforcement part 35 may run in a direction parallel to the outer edge of the first plate 30. In addition, the first plate 30 may include both the reinforcement part 35 running from the first center part 33 toward the outer edge of the first plate 30, and the reinforcement part 35 running in a direction parallel to the outer edge of the first plate 30.

A width W1 of the reinforcement part 35 is, for example, 1 mm or more, may be 3 mm or more, or may be 5 mm or more. In addition, the width W1 of the reinforcement part 35 is, for example, 20 mm or less, may be 15 mm or less, or may be 10 mm or less.

Next, a layer structure of the first plate 30 is described with reference to Fig. 6. The first plate 30 comprises at least a base layer 63. The base layer 63 is a layer that occupies most of the first extension part 34 of the first plate 30. A thickness of the base layer 63 is, for example, 50% or more of the thickness A1 of the overall first plate 30, may be 60% or more, or may be 70% or more. The elastic restorability of the first extension part 34 mainly depends on a property of the base layer 63.

The base layer 63 contains a biocompatible material having suitable elasticity. For example, the base layer 63 contains fluororesins, polyesters, polyamides, urethanes, silicones, polyetherketones, or other biocompatible polymers, and combinations thereof.

Examples of fluororesins include polytetrafluoroethylene (PTFE), tetrafluoroethylene hexafluoropropylene copolymer (FEP), tetrafluoroethylene ethylene copolymer (ETFE), tetrafluoroethylene ethylene copolymer (PVDF), etc.

Examples of polyesters include polyethylene terephthalate, polyester-based elastomers (TPEE), etc. Elastomer is a material which is soft and fluid when heated, and returns to a rubbery state when cooled.

Examples of polyamides include PC12, polyamide elastomers (TPAE), etc..

Urethanes are compounds with urethane bonds formed by reaction of alcohols and isocyanates. Examples of urethanes include polyurethane elastomers (TPU), etc..

Silicones are silicone rubber (SR), etc., for example.

Examples of polyetherketones include polyetheretherketone (PEEK), etc.

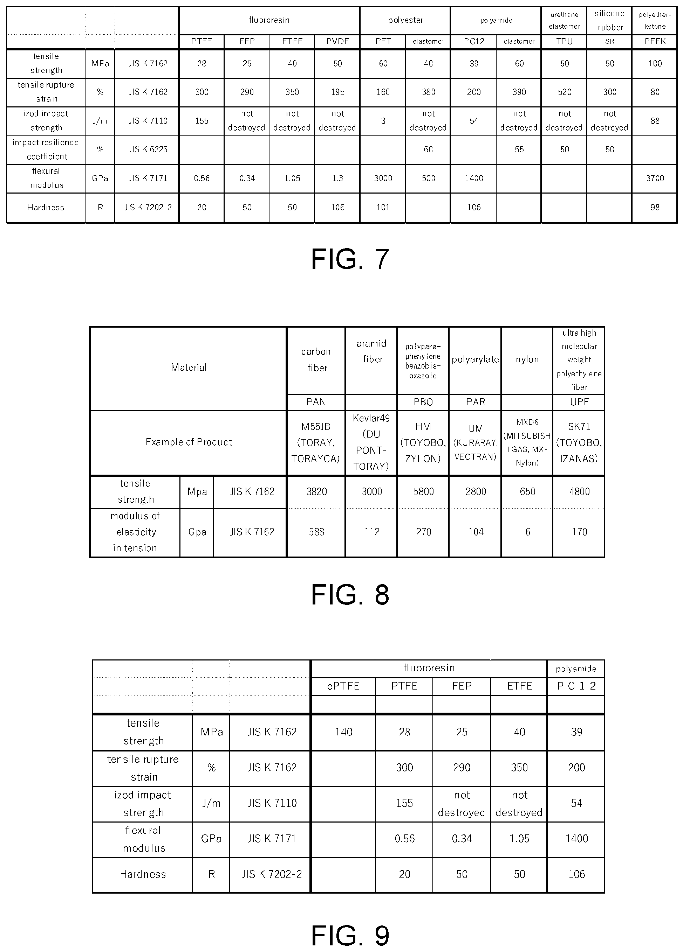

Fig. 7 shows examples of materials forming the base layer 63 and mechanical properties of the respective materials.

As shown in Fig. 6, the first plate 30 may include a first surface layer 64 forming the contact surface 31. The first surface layer 64 may be stacked on the base layer 63. The first surface layer 64 may be formed of a biocompatible or biodegradable material. This can suppress elimination reaction of a living body to the first plate 30. The first surface layer 64 may preferably contain fluororesin. This can improve the adhesiveness of the first plate 30 to the tissue.

Examples of biocompatible materials are the following biocompatible polymers.
ePTFE (porous material), fluororesin such as PTFE, polyamides, urethane-based polymers, PEEK

Examples of biodegradable materials are the following biodegradable polymers.
Lactide cabrolactan copolymers, ePTFE+ [collagen/extracellular matrix]

As shown in Fig. 6, the first plate 30 may include a second surface layer 65 forming the non-contact surface 32. The second surface layer 65 may be stacked on the base layer 63. The second surface layer 65 is preferably formed such that a coefficient of friction between the second surface layer 65 and the catheter 11 is low. This allows the closure device 20 to be smoothly delivered inside the catheter 11.

For example, the second surface layer 65 contains fluororesins, polyesters, polyamides, urethanes, or other biocompatible polymers, and combinations thereof.

Examples of fluororesins include ePTFE, PTFE, FEP, ETFE, etc.

Examples of polyamides include PC12, polyamide elastomers (TPAE), etc.

Fig. 8 shows examples of materials forming the second surface layer 65 and mechanical properties of the respective materials.

As shown in Fig. 6, the reinforcement part 35 may include a bundle of fibers 66. The fibers 66 run along a direction in which the reinforcement part 35 runs in a plan view. The fibers 66 are embedded in the base layer 63, for example.

The fiber 66 preferably has a tensile strength higher than the base layer 63. Examples of the fibers 66 include carbon fibers, aramid fibers, polyparaphenylene benzobisoxazole, polyarylate, nylon, ultra high molecular weight polyethylene fibers, etc.

Fig. 9 shows examples of materials forming the fiber 66 and mechanical properties of the respective materials.

A height H1 of the reinforcement part 35 is, for example, 1 mm or more, may be 3 mm or more, and may be 5 mm or more. In addition, the height H1 of the reinforcement part 35 is, for example, 20 mm or less, may be 15 mm or less, or may be 10 mm or less.

Preferable mechanical properties of the first extension part 34 are described.

A tensile strength of the first extension part 34 is, for example, 20 MPa or more, may be 25 MPa or more, or may be 30 MPa or more. In addition, the tensile strength of the first extension part 34 is, for example, 150 MPa or less, may be 130 MPa or less, may be 100 MPa or less, or may be 80 MPa or less.

A tensile rupture strain of the first extension part 34 is, for example, 50% or more, may be 80% or more, may be 100% or more, or may be 150% or more. In addition, the tensile rupture strain of the first extension part 34 is, for example, 550% or less, may be 450% or less, may be 400% or less, or may be 300% or less.

The tensile strength and the tensile rupture strain of the first extension part 34 are measured in line with JIS K 7161. As shown by one-dot chain lines indicated by a reference numeral 30S in Fig. 5, for example, a measurement sample is obtained by cutting a portion of the first extension part 34 along a direction from the first center part 33 toward the outer edge of the first plate 30.

A layer structure of the waist part 50 may be entirely the same as the layer structure of the first plate 30, may be partially the same, or may be different therefrom. For example, the waist part 50 may include a layer integrally formed by the same material as that of the base layer 63 of the first plate 30.

As shown in Fig. 6, the closure device 20 according to the invention comprises an IC chip 36 embedded in the resin of the closure device 20. The IC chip 36 includes, for example, a MEMS.

The IC chip 36 has a sensor function, for example. For example, the IC chip 36 can function as a heart rate sensor that electrically measures pulses of the heart 1.

The IC chip 36 may have a radar function. For example, the IC chip 36 may include a transceiver that transmits and receives radio waves. This allows an object around the closure device 20 to be detected.

The IC chip 36 may have a function of detecting sound. For example, the IC chip 36 may include a sound pickup microphone that detects sound, and a converter that converts detected sound to an electric signal.

Since the IC chip 36 is embedded in the resin, detachment of the IC chip 36 from the closure device 20 can be suppressed. Data obtained by the IC chip 36 are transmitted to an external apparatus by wireless communication or the like.

The IC chip 36 includes, according to the invention, a power generating element 36a. The power generating element 36a generates electric power using pulses of the heart 1, for example. The use of the power generating element 36a allows a continuous operation of the IC chip 36 over a long period of time.

### (Manufacturing Method of Closure Device)

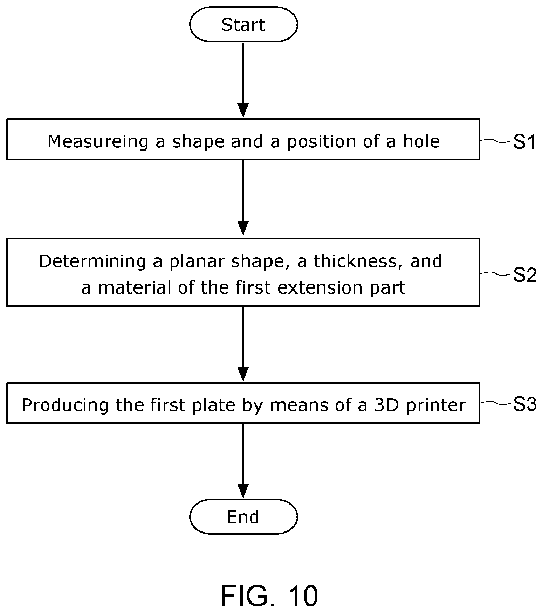

Next, a manufacturing method of the closure device 20 is described with reference to Fig. 10. First, a measuring step S1 that measures a shape and a position of a hole or duct in a tissue related to a cardiac disease is performed. A shape and a position of the arterial duct 73 are measured by echo, etc., for example.

Following thereto, a producing step that produces the closure device 20 is performed based on the information on the shape and the position of the arterial duct 73. The producing step may include a step of designing and producing a part of the closure device 20 in accordance with the measuring result of the shape and the position of the arterial duct 73. For example, as shown in Fig. 10, the producing step may include a first plate designing step S2 and a first plate producing step S3. This can provide the closure device 20 comprising the first plate 30 which is more suited to individual cases of patients. A predetermined design or a previously produced component may be used for a part of the closure device 20, which is less dependent on individual cases.

In the first plate designing step S2, design information of the first extension part 34 may be generated based on a learning model. For example, the design information of the first extension part 34 can be obtained by inputting the shape and the position of the arterial duct 73, which are obtained in the measuring step S1, to a computer comprising a learning model. The design information includes at least one of a planar shape, a thickness, and a material of the first extension part 34. The design information may include any two of the planar shape, the thickness, and the material of the first extension part 34. The design information may include the planar shape, the thickness, and the material of the first extension part 34.

A learning model of the first plate 30 can be obtained by means of a computer trained by a training data. The training data includes shapes and positions of holes or ducts in tissues of patients having cardiac disease as input objects. The training data includes the aforementioned design information of the first extension part 34 as output values. Input to the learning model may further include other information such as an inner diameter of the catheter 11, a coefficient of friction of the catheter 11, etc.

Input to the computer comprising the learning model may include physical characteristics of a patient, such as a height, a weight, etc. Input to the computer comprising the learning model may include information such as patient's age, gender, etc. The computer may output the design information based on prediction of future changes that may occur in a tissue such as a heart. For example, when a patient is determined as a child from the input information, the computer may output the design information based on prediction of growth that may occur in a tissue of a heart, etc. This allows the closure device 20 to properly function after the patient has grown.

The first plate producing step S3 may produce the first plate 30 by means of a 3D printer. This allows the closure device 20 to be quickly produced based on the information obtained in the measuring step S1.

The producing step may include a waist part designing step and a waist part producing step. Similarly to the first plate designing step S2, the waist part designing step may generate design information of the waist part 50 based on a learning model. In this case, the learning model may use, as an input, information on a thickness in the tissue of the aorta 71, etc. Similarly to the first plate producing step S3, the waist part producing step may produce the waist part 50 by means of a 3D printer.

In a treatment, such as ASO, which uses a conventional closure device, a closure device suited to a patient is selected depending on a shape of a cardiac disease of a patient. For example, suppose that lineups of a conventional closure device prepare closure devices every 1 mm in diameter. When a diameter of a hole of a cardiac disease is 19. 3 mm, a closure device having a diameter of 20 mm is used. Namely, a ready-made closure device is used. This may result in erosion because of the excessive dimension of the closure device.

On the other hand, when the closure device 20 is produced by means of a 3D printer, the closure device 20 can have a shape corresponding to a shape of a cardiac disease of a patient. Namely, a custom-made closure device 20 can be provided. Thus, there is less possibility that the closure device 20 has an excessive dimension. This can suppress a problem such as erosion. A resolution of a 3D printer is, for example, 500 µm or less. Thus, the dimension of the closure device 20 can have an accuracy of 500 µm. The resolution of a 3D printer may be 300 µm or less, may be 100 µm or less, or may be 50 µm or less.

The producing step such as the first plate designing step may be realized by a software running on a computer. By installing a program in a computer, for example, the computer may execute the first plate designing step using a learning model.

The program may be pre-installed on a computer when the computer is shipped. Alternatively, the program may be installed on the computer after it has been shipped by using a computer readable non-transitory storage medium in which the program is stored. A type of the storage medium is not particularly limited, and various types may be considered. Namely, the storage medium may be a portable storage medium such as a magnetic disk or an optical disk, a fixed storage medium such as a hard disk and a memory, etc. The program may be distributed via a communication line such as an internet. When the program is distributed via a communication line, a storage medium storing the program according to this embodiment is at least temporarily present on a server for distribution.

### (Using Method of Closure Device)

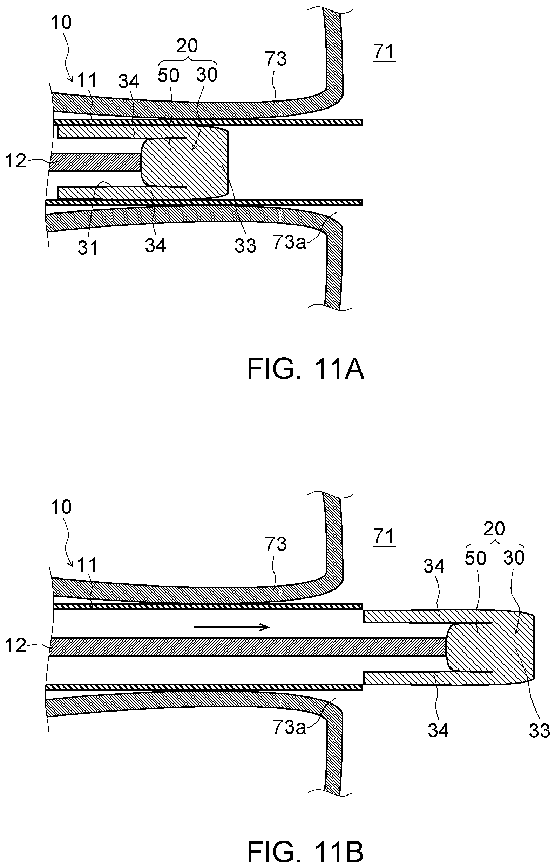

Next, a using method of the closure device 20 is described with reference to Figs. 11A to 11D.

First, the catheter 11 is inserted into a body. Then, as shown in Fig. 11A, the catheter 11 is moved until the distal end of the catheter 11 reaches the aorta 71 through the arterial duct 73. In addition, the delivery cable 12 is attached to the waist part 50 of the closure device 20, and the closure device 20 is inserted into the catheter 11. Then, as shown in Fig. 11A, the closure device 20 is moved closer to the distal end of the catheter 11. The closure device 20 is positioned inside the catheter 11, with at least the first plate 30 being folded.

As shown in Fig. 11A, when the closure device 20 is positioned inside the catheter 11, the waist part 50 may be compressed in the radial direction by a force received from the inner wall of the catheter 11. The compressed waist part 50 has a dimension smaller than the internal diameter of the catheter 11 in the radial direction. The radial direction means a direction from the first center part 33 toward the outer edge of the first extension part 34, in a state where the first extension part 34 is extended around the first center part 33. After having been taken out from the catheter 11, the waist part 50 preferably has a dimension larger than the internal diameter of the catheter 11 in the radial direction.

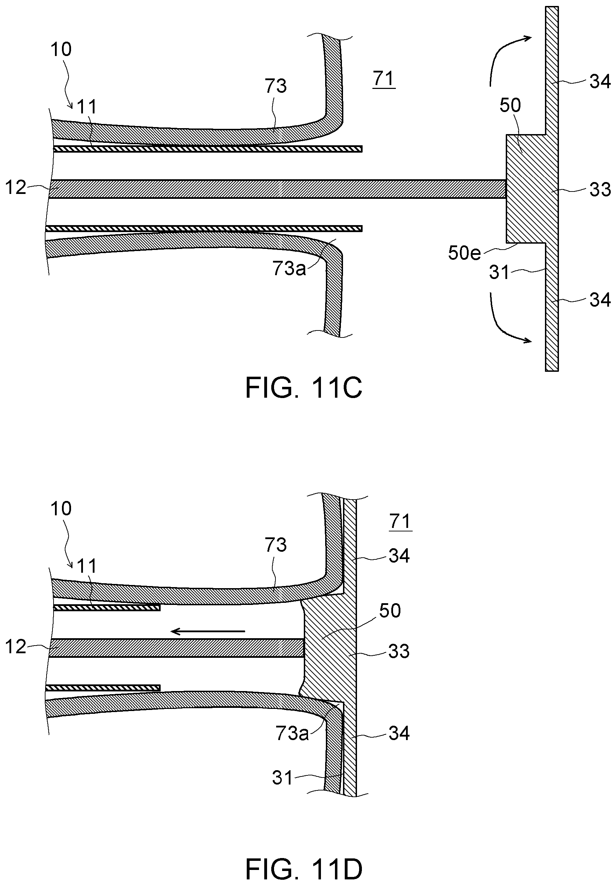

As shown in Fig. 11B, the closure device 20 is further moved so that the first plate 30 of the closure device 20 is pushed out from the catheter 11. Then, as shown in Fig. 11C, the first extension part 34 elastically restores, i.e., unfolds to extend around the first center part 33. Since the waist part 50 is released from the force received from the catheter 11, it can expand in the radial direction.

Following thereto, as shown in Fig. 11D, the delivery cable 12 is retracted so that the contact surface 31 of the first plate 30 comes into contact with the wall surface of the aorta 71. The waist part 50 also comes into contact with the wall surface of the arterial duct 73. Thus, the arterial duct 73 can be closed. Thereafter, the delivery cable 12 is detached from the closure device 20. In addition, the catheter 11 together with the delivery cable 12 is pulled out from the body.

### (Effect of Embodiment)

In this embodiment, the first plate 30 of the closure device 20 comprises the first extension part 34 containing a resin having elastic restorability. Thus, the first plate 30 of the closure device 20, which is delivered in the folded state, can elastically restore to its extending shape. Thus, the first plate 30 can close the arterial duct 73. As compared with the use of a conventional mesh structure, the first plate 30 has a higher degree of freedom in the planar shape, and is easy to manufacture. For example, the first plate 30 having a desired shape can be quickly produced by means of a 3D printer. Thus, the first plate 30 suited for various shapes of the arterial duct 73 can be easily provided. This can suppress generation of erosion, which may be caused by an excessive dimension of the closure device 20.

This embodiment can close a cardiac disease of the arterial duct 73 or the like without using a mesh structure made of metal wires. Since the closure device 20 includes no mesh structure, generation of erosion, which may be caused by damaged metal wire, can be suppressed.

By suppressing erosion, dangerous conditions such as cardiac tamponade can be suppressed.

The above embodiment can be variously modified. Modification examples are described below, with reference to the drawings according to need. In the following description and the drawings used in the following description, the same reference numeral as that used for the part that can be structure in the same way as in the above embodiment, and redundant description is omitted. In addition, when the effect obtained in the above embodiment is obtained in the modification examples, its description may be omitted.

### (Modification Example of Closure System)

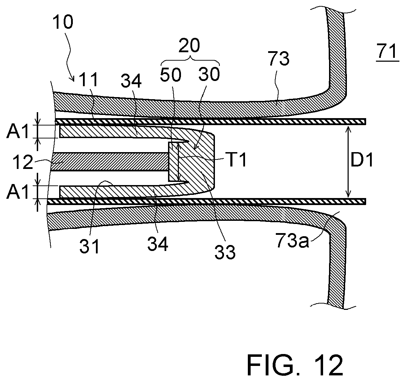

Fig. 12 is a view showing an example of the closure system 10. The catheter 11 has an internal diameter D1 [mm]. The internal diameter D1 may be larger than 2×A1+T1. A1 is a thickness of the first extension part 34 of the first plate 30. T1 is a maximum dimension of the waist part 50 in a plan view. When the internal diameter D1 is larger than 2×A1+T1, compression of the waist part 50 in the radial direction of the catheter 11 can be suppressed. This can reduce a frictional force between the inner wall of the catheter 11 and the closure device 20, whereby the closure device 20 can be easily moved inside the catheter 11. This allows the closure device 20 to be delivered quickly to the tissue of the body.

### (Modification Example of Cardiac Disease)

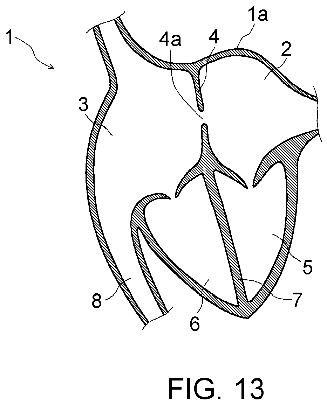

The above embodiment shows the example in which the patent ductus arteriosus is treated using the closure device 20. However, the cardiac disease in which the closure device 20 is used is not limited to the patent ductus arteriosus. For example, another cardiac disease, such as atrial septal defect, ventricular septal defect, patent foramen ovale, etc., may be treated using the closure device 20. Fig. 13 is a view showing an example of a cardiac disease of the heart 1.

The heart 1 includes a left atrium 2, a right atrium 3, an atrial septum 4, a left ventricle 5, a right ventricle 6, and a interventricular septum 7, which are positioned inside an atrial wall 1a. The atrial septum 4 is positioned between the left atrium 2 and the right atrium 3. The interventricular septum 7 is positioned between the left ventricle 5 and the right ventricle 6. In the example shown in Fig. 13, the atrial septum 4 has a hole 4a. Namely, the cardiac disease of the heart 1 is ventricular septal defect.

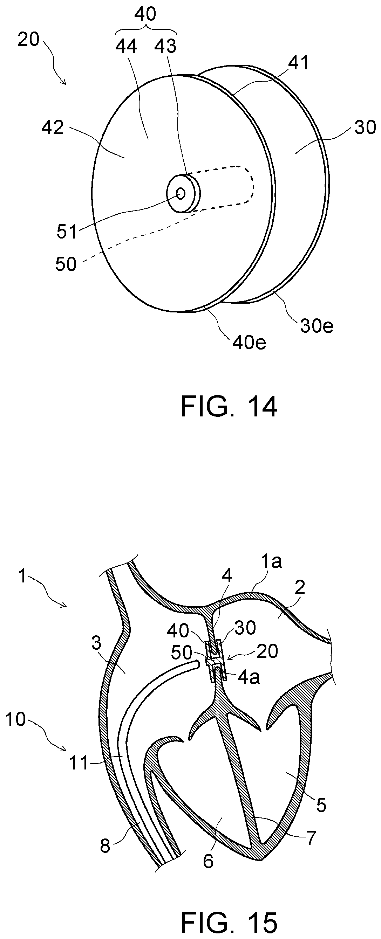

Fig. 14 is a view showing an example of the closure device 20. As shown in Fig. 14, the closure device 20 may comprise, in addition to the first plate 30 and the waist part 50, a second plate 40 connected to the waist part 50. The second plate 40 is arranged inside the heart 1 such that the hole 4a of the atrial septum 4 is sandwiched between the second plate 40 and the first plate 30. Fig. 15 is a view showing that the hole 4a of the atrial septum 4 is closed by the closure device 20 of Fig. 14.

As shown in Fig. 14, the second plate 40 includes a contact surface 41 and a non-contact surface 42. The contact surface 41 faces the contact surface 31 of the first plate 30. The contact surface 41 may contact the atrial septum 4. The non-contact surface 42 is a surface opposite the contact surface 41.

As shown in Fig. 14, the second plate 40 has a second center part 43, and a second extension part 44 extending around the second center part 43. The second center part 43 is connected to the waist part 50. The second center part 43 may be integral with the waist part 50. The second extension part 44 may be integral with the second center part 43.

Similarly to the first extension part 34, the second extension part 44 contains a resin having elastic restorability. Thus, the second extension part 44 can return from a state in which the second extension part 44 is folded such that surface portions of the second extension part 44 face to each other, to a state in which the second extension part 44 is extended around the second center part 43.

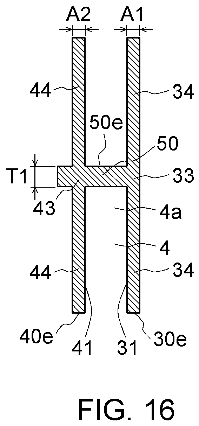

Fig. 16 is a view illustrating dimensions of the closure device 20 of Fig. 14. The second extension part 44 has a thickness A2 [mm]. The second extension part 44 has a maximum dimension B2 [mm] in a plan view. When the second plate 40 is circular, the maximum dimension B2 is a diameter of the second plate 40. A2/B2 is, for example, 1/50 or more, may be 1/30 or more, or may be 1/20 or more. A2/B2 may be 1/3 or less, may be 1/5 or less, or may be 1/10 or less.

An outer edge 40e of the second plate 40 is preferably free of hard material such as a metal. For example, the outer edge 40e is preferably formed of a resin such as the aforementioned biocompatible material. This can suppress generation of erosion in the tissue, which may be caused by contact between the tissue and the outer edge 40e.

A layer structure of the second extension part 44 may be similar to the layer structure of the first extension part 34. For example, the second extension part 44 comprises at least the aforementioned base layer 63. The second extension part 44 may include the aforementioned first surface layer 64 forming the contact surface 41. In addition, the second extension part 44 may include the aforementioned second surface layer 65 forming the non-contact surface 42.

Since preferable mechanical properties of the second extension part 44 are similar to those of the first extension part 34, description is omitted.

Next, a manufacturing method of the closure device 20 of Fig. 14 is described. Also in this modification example, a measuring step S1 that measures a shape and a position of the hole 4a in the tissue is performed. At this time, a thickness of the tissue, e.g., a thickness of the atrial septum 4 may further be measured.

Following thereto, a producing step that produces the closure device 20 is performed based on the information on the shape and the position of the hole 4a. The producing step may include the aforementioned first plate designing step S2 and the first plate producing step S3. In addition, the producing step may include a second plate designing step and a second plate producing step.

Similarly to the first plate designing step S2, the second plate designing step may generate design information of the second extension part 44 based on a learning model. For example, the design information of the second extension part 44 can be obtained by inputting the shape and the position of the arterial duct 73, which are obtained in the measuring step S1, to a computer comprising a learning model. The design information includes at least one of a planar shape, a thickness, and a material of the second extension part 44. The design information may include any two of the planar shape, the thickness, and the material of the second extension part 44. The design information may include the planar shape, the thickness, and the material of the second extension part 44.

A learning model of the second plate 40 can be obtained by means of a computer trained by a training data. The training data includes shapes and positions of holes or ducts in tissues of patients having cardiac disease as input objects. The training data includes the aforementioned design information of the second extension part 44 as output values. Input to the learning model may further include other information such as an inner diameter of the catheter 11, a coefficient of friction of the catheter 11, etc.

Similarly to the first plate producing step S3, the second plate producing step may produce the second plate 40 by means of a 3D printer.

The producing step may include a waist part designing step and a waist part producing step. Similarly to the first plate designing step S2, the waist part designing step may generate design information of the waist part 50 based on a learning model. In this case, the learning model may use, as an input, information of a thickness of the tissue such as the atrial septum 4. Similarly to the first plate producing step S3, the waist part producing step may produce the waist part 50 by means of a 3D printer.

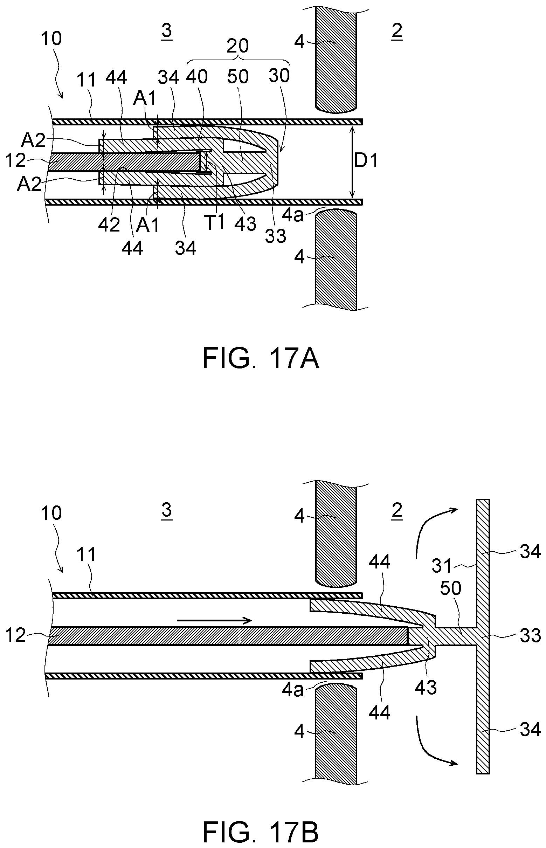

Next, a using method of the closure device 20 of Fig. 14 is described with reference to Figs. 17A to 17D.

First, the catheter 11 is inserted from a blood vessel at the base of a leg. Then, as shown in Fig. 17A, the catheter 11 is moved until the distal end of the catheter 11 reaches the left atrium 2 through the hole 4a of the atrial septum 4. In addition, the delivery cable 12 is attached to the waist part 50 of the closure device 20, and the closure device 20 is inserted into the catheter 11. Then, as shown in Fig. 17A, the closure device 20 is moved closer to the distal end of the catheter 11. The closure device 20 is positioned inside the catheter 11, with the first plate 30 and the second plate 40 being folded.

The internal diameter D1 of the catheter 11 may be larger than 2×A1+2×A2+T1. A1 is a thickness of the first extension part 34 of the first plate 30. A2 is a thickness of the second extension part 44 of the second plate 40. T1 is a maximum dimension of the waist part 50 in a plan view. When the internal diameter D1 is larger than 2×A1+2×A2+T1, compression of the waist part 50 in the radial direction of the catheter 11 can be suppressed. This can reduce a frictional force between the inner wall of the catheter 11 and the closure device 20, whereby the closure device 20 can be easily moved inside the catheter 11.

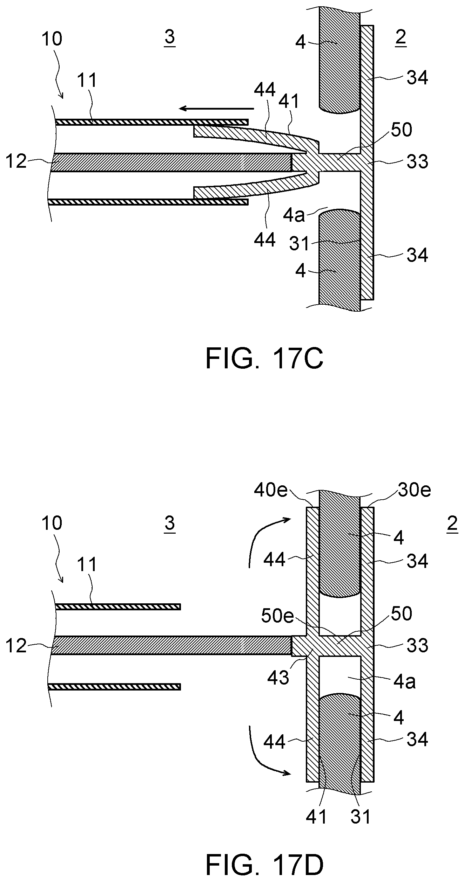

As shown in Fig. 17B, the closure device 20 is further moved so that the first plate 30 of the closure device 20 is pushed out from the catheter 11. Then, as shown in Fig. 17C, the extension part 34 elastically restores, i.e., unfolds to extend around the first center part 33.

Following thereto, as shown in Fig. 17C, the delivery cable 12 is retracted so that the contact surface 31 of the first plate 30 comes into contact with the atrial septum 4. Then, the catheter 11 is retracted so that the second plate 40 is discharged from the catheter 11. Then, as shown in Fig. 17D, the second extension part 44 elastically restores, i.e., unfolds to extend around the second center part 43. Thus, the atrial septum 4 can be sandwiched between the first plate 30 and the second plate 40. This can more securely close the hole 4a in the atrial septum 4. In addition, detachment of the closure device 20 from the tissue can be suppressed.

As compared with the use of a conventional mesh structure, the closure device 20 in this modification example also has a higher degree of freedom in the planar shape, and is easy to manufacture. For example, the first plate 30 and the second plate 40 each having a desired shape can be quickly produced by means of a 3D printer. Thus, the first plate 30 and the second plate 40 which are suited for various shapes of the hole 4a of the atrial septum 4 can be easily provided. This can suppress generation of erosion, which may be caused by an excessive dimension of the closure device 20. In addition, since the closure device 20 includes no mesh structure, generation of erosion, which may be caused by damaged metal wire, can be suppressed.

### (Modification Example of Closure System)

When the closure device 20 is positioned inside the catheter 11, the waist part 50 of the closure device 20 comprising the first plate 30 and the second plate 40 may be compressed in the radial direction, although not shown. Namely, the internal diameter D1 of the catheter 11 may be smaller than 2×A1+2×A2+T1. In this case, similarly to the example shown in Fig. 11D, after having been pushed out from the catheter 11, the waist part 50 of the closure device 20 may be in contact with the wall surface of the hole 4a.

### (Modification Example of Hole Position)

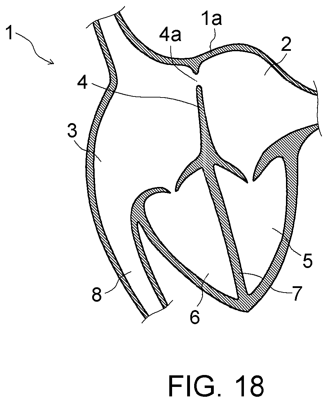

An example in which the hole 4a is positioned close to the atrial wall 1a, as shown in Fig. 18, is described. When a conventional closure device formed of a mesh structure is used, a part of the closure device contacts the atrial wall 1a. Thus, there is a possibility that the closure device cannot appropriately close the hole 4a.

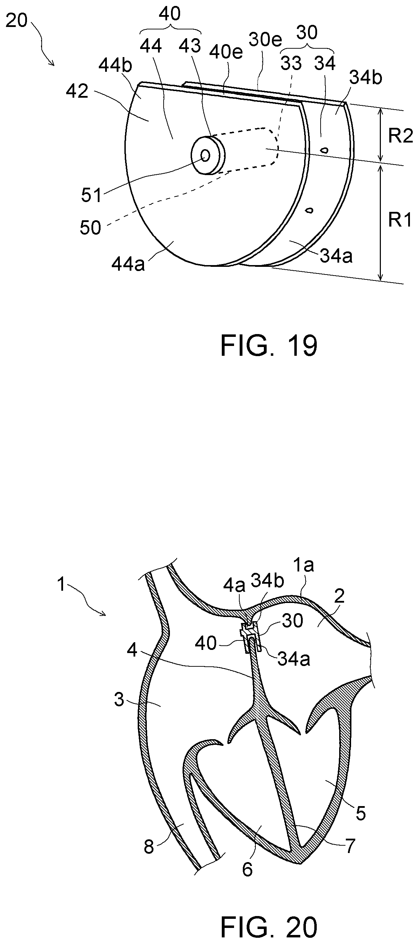

On the other hand, since the first plate 30 has a high degree of freedom in the planar shape, this patent application can provide a closure device 20 as shown in Fig. 19, for example. In the example shown in Fig. 19, the first extension part 34 of the first plate 30 has a first portion 34a, and a second portion 34b having a shape asymmetric to the first portion 34a with respect to a center of the first center part 33. For example, the first portion 34a is semicircular, while the second portion 34b has a shape obtained by cutting a part of a semicircular arc along a line parallel to the diameter. By machining the second portion 34b in this manner, contact between the first plate 30 and the atrial wall 1a of the heart 1 can be suppressed, as shown in Fig. 20. Thus, the closure device 20 can appropriately close the hole 4a. By suppressing contact with the atrial wall 1a, generation of erosion in the atrial wall 1a can be suppressed.

In Fig. 19, reference numerals R1 and R2 respectively represent a dimension of the first portion 34a and a dimension of the second portion 34b in a direction along which they are arranged side by side. The dimension R2 of the second portion 34b is smaller than the dimension R1 of the first portion 34a. The dimension R2 is, for example, 9/10 or less of the dimension R1, may be 8/10 or less, or may be 7/10 or less.

Similarly to the first plate 30, the second plate 40 has a high degree of freedom in the planar shape. As shown in Fig. 19, the second extension part 44 of the second plate 40 may include a third portion 44a, and a fourth portion 44b having a shape asymmetric to the first portion 44a with respect to a center of the second center part 43. For example, the third portion 44a is semicircular, while the fourth portion 44b has a shape obtained by cutting a part of a semicircular arc along a line parallel to the diameter. By machining the fourth portion 44b in this manner, contact between the second plate 40 and the atrial wall 1a of the heart 1 can be suppressed, as shown in Fig. 20. Thus, the closure device 20 can appropriately close the hole 4a. By suppressing contact with the atrial wall 1a, generation of erosion in the atrial wall 1a can be suppressed.

### (Modification Example of Manufacturing Method of Closure Device)

Although the above embodiment shows the example in which the closure device 20 is designed based on a learning model, the closure device 20 may be designed using another method. For example, a person may design the closure device 20 based on experience.

In addition, although the above embodiment shows the example in which the closure device 20 is produced by means of a 3D printer, the closure device 20 may be produced using another method. For example, the closure device 20 may be produced by a molding method such as sheet molding.

### (Modification Example of Cardiac Disease)

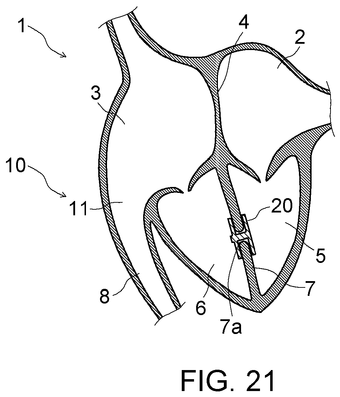

The above embodiment shows the example in which the ventricular septal defect is treated using the closure device 20. However, the cardiac disease in which the closure device 20 is used is not limited to the ventricular septal defect. For example, another cardiac disease, such as atrial septal defect, patent ductus arteriosus, patent foramen ovale, etc., may be treated using the closure device 20. Fig. 21 is a view showing an example in which a hole 7a in the interventricular septum 7 is closed using the closure device 20.

### (Modification Example of Closure Device)

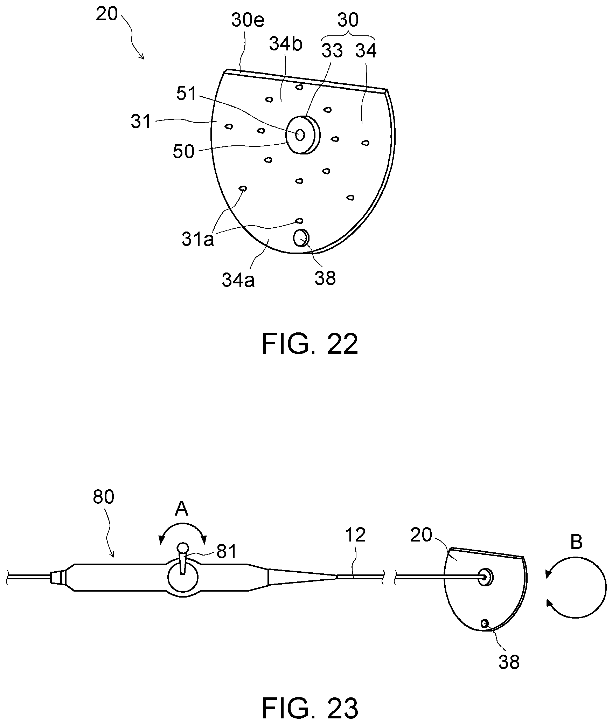

Fig. 22 is a view showing an example of the closure device 20. The first plate 30 does not have rotational symmetry. For example, similarly to the example of Fig. 19, the closure device 20 has a first portion 34a, and a second portion 34b having a shape asymmetric to the first portion 34a with respect to the center of the first center part 33. In this case, in order to appropriately close a hole or duct of a cardiac disease, it is necessary to control positions of the first portion 34a and the second portion 34b. In order to appropriately perform the control, it is necessary to monitor the positions of the first portion 34a and the second portion 34b, while the closure device 20 is being inserted in a body.

In this modification example, the first plate 30 includes a first marker 38. The first marker 38 is positioned on the first portion 34a, for example. While the closure device 20 is being inserted in the body, the position of the first portion 34a can be determined by detecting the first marker 38 by means of echo or the like. Thus, a rotational angle of the first plate 30 can be calculated, for example.

The first marker 38 can have any structure, as long as the position of the marker 38 can be detected from outside the body by an inspection method such as echo. For example, the first marker 38 may have a structure raised from a surface of the first plate 30. Alternatively, the first marker 38 may have a structure recessed from the surface of the first plate 30. The first marker 38 may contain a material, such as a metal, which is different from that of an element surrounding the first marker 38.

Fig. 23 is a view showing an example of a handle 80 for operating the closure device 20. The handle 80 includes a lever 81 for controlling a rotational angle of the closure device 20. For example, by moving the lever 81 in a rotational direction A, the closure device 20 can be rotated in a rotational direction B. A rotational angle of the closure device 20 can be controlled by operating the lever 81 while monitoring a rotational angle of the closure device 20 by means of the marker 38.

Any driving mechanism for rotating the closure device 20 will do. For example, the closure device 20 may be rotated by rotating the delivery cable 12 or the catheter 11.

Although not shown, the closure device 20 may include a plurality of markers.

For example, the first plate 30 may include, in addition to the first marker 38 positioned on the first portion 34a, a second marker positioned on the second portion 34b. The first marker 38 and the second marker may be different from each other in shape, dimension, material, etc.

Alternatively, the first plate 30 may include 12 markers that are equidistantly arranged along a circumference, similarly to hour marks on a clock.

Fig. 22 shows the example in which the first maker 38 is positioned on the contact surface 31 of the first plate 30. However, the position of the first marker 38 is not limited thereto, and the first marker 38 may be positioned on the non-contact surface 32 of the first plate 30. The first marker 38 may be positioned on the contact surface 41 or the non-contact surface 42 of the second plate 40.

Although not shown, an IC chip, a microactuator, a marker, etc., may be provided on a component of the closure system 10, which is other than the closure device 20. For example, the catheter 11, the delivery cable 12, etc., may include an IC chip, a microactuator, a marker, etc.

### (Modification Example of Using Method of Closure Device)

The above embodiment shows the example in which the closure device 20 is delivered into the heart 1 through the catheter 11. However, the means for delivering the closure device 20 to the heart 1 is not limited to the catheter 11. For example, the hear 1 may be surgically cut, and the closure device 20 may be delivered into the heart 1 through an incision. In this case, the closure device 20 may be sutured to the tissue using a thread and a needle.

In this modification example, since the first extension part 34 has the elastic restorability, the closure device 20 can be passed through the incision, with the first extension part 34 being in the folded state. In addition, the first extension part 34 can be easily adhered to the tissue by means of the elasticity of the first extension part 34. In addition, since the first extension part 34 has high degree of freedom in the planar shape, a hole or duct in a tissue related to a cardiac disease can be appropriately closed.

Some modification examples on the above embodiment have been described, it goes without saying that the modification examples can be suitably combined and applied.

- 1: Heart
- 1a: Atrial wall
- 2: Left atrium
- 3: Right atrium
- 4: Atrial septum
- 4a: Hole
- 5: Left ventricle
- 6: Right ventricle
- 7: Interventricular septum
- 7a: Hole
- 8: Lower great vein
- 10: Closure system
- 11: Catheter
- 12: Delivery cable
- 20: Closure device
- 30: First plate
- 31: Contact surface
- 31a: Projection
- 32: Non-contact surface
- 33: First center part
- 34: First extension part
- 34a: First portion
- 34b: Second portion
- 35: Reinforcement part
- 36: IC chip
- 36a: Power generating element
- 38: Marker
- 40: Second plate
- 41: Contact surface
- 42: Non-contact surface
- 43: Second center part
- 44: Second extension part
- 50: Waist part
- 51: Joint
- 63: Base layer
- 64: First surface layer
- 65: Second surface layer
- 66: Fiber
- 71: Aorta
- 72: Pulmonary artery
- 73: Arterial duct
- 80: Handle
- 81: Lever

## Claims

1. A closure system for closing a hole or duct in a tissue related to a cardiac disease, comprising:
a catheter (11) having an internal diameter D1 in mm; and
a closure device (20) to be delivered to the hole or duct in the tissue through the catheter (11);
wherein:
the closure device (20) comprises: a first plate (30) including a first center part (33), and a first extension part (34) extending around the first center part (33) and containing a resin having elastic restorability; and a waist part (50) connected to the first center part (33);
the elastic restorability is a property by which the first extension part (34) returns from a state in which the first extension part (34) is folded such that surface portions of the first extension part (34) face to each other, to a state in which the first extension part (34) is extended around the first center part (33);
the first extension part (34) has a thickness A1 in mm;
the waist part (50) has a maximum dimension T1 in mm in a plan view; and
**characterized in that**
the internal diameter D1 of the catheter (11) is larger than 2×A1+T1,
the closure system comprising an IC chip (36) embedded in the resin,
wherein the IC chip (36) includes a power generating element (36a) that is configured to generate electric power using pulses of a heart.

2. The closure system according to claim 1, wherein
the first extension part (34) has a maximum dimension B1 in mm in a plan view, and
A1/B1 is 1/50 or more.

3. The closure system according to claim 1 or 2, wherein
the first plate (30) includes a base layer (63) extending around the first center part (33) and containing fluororesin, polyester, polyamide, urethane, silicone, or polyetherketone, and
a thickness of the base layer (63) is 50% or more of the thickness A1 of the overall first plate (30).

4. The closure system according to any one of claims 1 to 3, wherein
the first extension part (34) includes, in a plan view, a first portion (34a), and a second portion (34b) having a shape asymmetric to the first portion (34a) with respect to a center of the first center part (33).

5. The closure system according to any one of claims 1 to 4, comprising a second plate (40) connected to the waist part (50), wherein
the second plate (40) comprises a second center part (43) connected to the waist part (50), and a second extension part (44) extending around the second center part (43) and containing a resin having elastic restorability.

6. The closure system according to claim 5, wherein
the second extension part (44) has a thickness A2 in mm,
and
the internal diameter D1 of the catheter (11) is larger than 2×A1+2×A2+T1.

7. The closure system according to any one of claims 1 to 6, comprising a handle (80) for operating the closure device.

8. The closure system according to claim 7, wherein
the closure device includes a marker (38) provided on the first plate (30), and
the handle (80) includes a lever (81) that controls a rotational angle of the first plate (30).

## Patentansprüche

1. Verschlusssystem zum Verschließen einer Öffnung oder eines Kanals in einem Gewebe im Zusammenhang mit einer Herzerkrankung, umfassend:
einen Katheter (11) mit einem Innendurchmesser D1 in mm; und
eine Verschlussvorrichtung (20), die durch den Katheter (11) zu dem Loch oder Kanal im Gewebe gebracht wird;
wobei
die Verschlussvorrichtung (20) umfasst: eine erste Platte (30) mit einem ersten Mittelteil (33) und einem ersten Verlängerungsteil (34), das sich um den ersten Mittelteil (33) herum erstreckt und ein Harz mit elastischer Rückstellfähigkeit enthält; und einen mit dem ersten Mittelteil (33) verbundenen Taillenteil (50);
die elastische Rückstellfähigkeit eine Eigenschaft ist, durch die der erste Verlängerungsteil (34) aus einem Zustand, in dem der erste Verlängerungsteil (34) so gefaltet ist, dass Oberflächenabschnitte des ersten Verlängerungsteils (34) einander zugewandt sind, in einen Zustand zurückkehrt, in dem der erste Verlängerungsteil (34) um den ersten Mittelteil (33) herum verlängert ist;
der erste Verlängerungsabschnitt (34) eine Dicke A1 in mm aufweist;
der Taillenabschnitt (50) in einer Draufsicht eine maximale Abmessung T1 in mm aufweist; und
**dadurch gekennzeichnet, dass**
der Innendurchmesser D1 des Katheters (11) größer ist als 2xA1+T1,
das Verschlusssystem einen in das Harz eingebetteten IC-Chip (36) umfasst,
wobei der IC-Chip (36) ein Stromerzeugungselement (36a) umfasst, das so konfiguriert ist, dass es unter Verwendung von Herzimpulsen elektrische Energie erzeugt.

2. Verschlusssystem nach Anspruch 1, wobei
der erste Verlängerungsabschnitt (34) in einer Draufsicht eine maximale Abmessung B1 in mm aufweist und
A1/B1 1/50 oder mehr beträgt.

3. Verschlusssystem nach Anspruch 1 oder 2, wobei
die erste Platte (30) eine Basisschicht (63) umfasst, die sich um den ersten Mittelteil (33) herum erstreckt und Fluorharz, Polyester, Polyamid, Urethan, Silikon oder Polyetherketon enthält, und
die Dicke der Basisschicht (63) 50 % oder mehr der Dicke A1 der gesamten ersten Platte (30) beträgt.

4. Verschlusssystem nach einem der Ansprüche 1 bis 3, wobei
der erste Verlängerungsteil (34) in einer Draufsicht einen ersten Abschnitt (34a) und einen zweiten Abschnitt (34b) umfasst, der in Bezug auf die Mitte des ersten Mittelteils (33) eine zum ersten Abschnitt (34a) asymmetrische Form aufweist.

5. Verschlusssystem nach einem der Ansprüche 1 bis 4, umfassend eine zweite Platte (40), die mit dem Taillenbereich (50) verbunden ist, wobei
die zweite Platte (40) einen zweiten Mittelteil (43), der mit dem Taillenteil (50) verbunden ist, und einen zweiten Verlängerungsteil (44) umfasst, der sich um den zweiten Mittelteil (43) herum erstreckt und ein Harz mit elastischer Rückstellfähigkeit enthält.

6. Verschlusssystem nach Anspruch 5, wobei
Der zweite Verlängerungsabschnitt (44) hat eine Dicke A2 in
mm, und
der Innendurchmesser D1 des Katheters (11) ist größer als 2xA1+2xA2+T1.

7. Verschlusssystem nach einem der Ansprüche 1 bis 6, umfassend einen Griff (80) zum Betätigen der Verschlussvorrichtung.

8. Das Verschlusssystem gemäß Anspruch 7, wobei
die Verschlussvorrichtung eine Markierung (38) umfasst, die auf der ersten Platte (30) vorgesehen ist, und
der Griff (80) einen Hebel (81) umfasst, der einen Drehwinkel der ersten Platte (30) steuert.

## Revendications

1. Système de fermeture destiné à fermer un trou ou un conduit dans un tissu lié à une maladie cardiaque, comprenant :
un cathéter (11) ayant un diamètre interne D1 en mm ; et
un dispositif de fermeture (20) destiné à être acheminé vers le trou ou le canal dans le tissu à travers le cathéter (11) ;
dans lequel :
le dispositif de fermeture (20) comprend : une première plaque (30) comprenant une première partie centrale (33) et une première partie d'extension (34) s'étendant autour de la première partie centrale (33) et contenant une résine ayant une capacité de restauration élastique ; et une partie de taille (50) reliée à la première partie centrale (33) ;
la capacité de restauration élastique est une propriété par laquelle la première partie d'extension (34) revient d'un état dans lequel la première partie d'extension (34) est pliée de telle sorte que les parties de surface de la première partie d'extension (34) se font face, à un état dans lequel la première partie d'extension (34) est étendue autour de la première partie centrale (33) ;
la première partie d'extension (34) a une épaisseur A1 en mm ;
la partie de taille (50) a une dimension maximale T1 en mm dans une vue en plan ; et
**caractérisé en ce que**
le diamètre interne D1 du cathéter (11) est supérieur à 2xA1+T1,
le système de fermeture comprenant une puce IC (36) intégrée dans la résine,
la puce IC (36) comprenant un élément générateur d'énergie (36a) qui est configuré pour générer de l'énergie électrique à partir des pulsations cardiaques.

2. Système de fermeture selon la revendication 1, dans lequel
la première partie d'extension (34) a une dimension maximale B1 en mm en vue en plan, et
A1/B1 est égal ou supérieur à 1/50.

3. Système de fermeture selon la revendication 1 ou 2, dans lequel
la première plaque (30) comprend une couche de base (63) s'étendant autour de la première partie centrale (33) et contenant de la résine fluorée, du polyester, du polyamide, de l'uréthane, du silicone ou du polyéthercétone, et
l'épaisseur de la couche de base (63) est égale ou supérieure à 50 % de l'épaisseur A1 de l'ensemble de la première plaque (30).

4. Système de fermeture selon l'une quelconque des revendications 1 à 3, dans lequel
la première partie d'extension (34) comprend, dans une vue en plan, une première partie (34a) et une deuxième partie (34b) ayant une forme asymétrique par rapport à la première partie (34a) par rapport au centre de la première partie centrale (33).

5. Système de fermeture selon l'une quelconque des revendications 1 à 4, comprenant une deuxième plaque (40) reliée à la partie de taille (50), dans lequel
la deuxième plaque (40) comprend une deuxième partie centrale (43) reliée à la partie de taille (50), et une deuxième partie d'extension (44) s'étendant autour de la deuxième partie centrale (43) et contenant une résine ayant une capacité de restauration élastique.

6. Système de fermeture selon la revendication 5, dans lequel
la deuxième partie d'extension (44) a une épaisseur A2 en
mm, et
le diamètre interne D1 du cathéter (11) est supérieur à 2xA1+2xA2+T1.

7. Système de fermeture selon l'une quelconque des revendications 1 à 6, comprenant une poignée (80) pour actionner le dispositif de fermeture.

8. Le système de fermeture selon la revendication 7, dans lequel
le dispositif de fermeture comprend un marqueur (38) prévu sur la première plaque (30), et
la poignée (80) comprend un levier (81) qui contrôle l'angle de rotation de la première plaque (30).
